Europäisches Patentamt

European Patent Office

Office européen des brevets

(19)

(11) Numéro de publication : **0 019 531**
**B1**

# (12) FASCICULE DE BREVET EUROPÉEN

(45) Date de publication du fascicule du brevet :
07.04.82

(21) Numéro de dépôt : 80400639.3

(22) Date de dépôt : 09.05.80

(51) Int. Cl.³ : **C 07 C 47/565**, C 07 C 45/54//
C07C59/52, C07C51/353,
C07C45/61, C07C47/575,
C07C45/68

(54) **Paraformylphénolate de sodium cristallisé, son procédé de préparation et son application.**

(30) Priorité : 14.05.79 FR 7912173

(43) Date de publication de la demande :
26.11.80 (Bulletin 80/24)

(45) Mention de la délivrance du brevet :
07.04.82 Bulletin 82/14

(84) Etats contractants désignés :
DE GB NL

(56) Documents cités :
FR - A - 751 687
FR - A - 2 132 364
US - A - 2 062 205
US - A - 3 673 257
CHEMICAL ABSTRACTS
Vol. 86, n° 25, 20 juin 1977, page 571,
réf. 189514k
Columbus, Ohio, US
CHEMICAL ABSTRACTS
Vol. 90, n° 19, 7 mai 1979, page 575,
réf. 151804z
Columbus, Ohio, US
CHEMICAL ABSTRACTS
Vol. 90, n° 7, 12 février 1979, page 584,
réf. 54666y
Columbus, Ohio, US

(73) Titulaire : SOCIETE FRANCAISE HOECHST Société
anonyme dite:
3, avenue du Général de Gaulle
F-92800 Puteaux (FR)

(72) Inventeur : Schouteeten, Alain
17, rue de Normandie
F-95460 Ezanville (FR)
Inventeur : Christidis, Yani
12, rue de Constantinople
F-75008 Paris (FR)

(74) Mandataire : Rinuy, Guy et al
Cabinet Rinuy et Santarelli 14, Avenue de la Grande
Armée
F-75017 Paris (FR)

Imprimerie Jouve, 18, rue St-Denis, 75001 Paris, France

**0 019 531**

« Paraformylphénolate de sodium cristallisé, son procédé de préparation et son application »

La présente invention concerne le paraformylphénolate de sodium cristallisé, son procédé de préparation industriel à partir de l'acide glyoxylique et son application.

Elle vise en particulier le paraformylphénolate de sodium cristallisé avec deux molécules d'eau.

Dans l'art antérieur, le paraformylphénolate de sodium est un dérivé du parahydroxybenzaldéhyde largement utilisé, soit pour accéder au parahydroxybenzaldéhyde, soit pour préparer des paraalcoxybenzaldéhydes divers, soit enfin pour effectuer des condensations variées engageant le groupement carbonyle aldéhydique.

Mais dans ces réactions, le paraformylphénolate de sodium n'est qu'un intermédiaire non isolé à l'état cristallisé pur.

A la connaissance de la Demanderesse, il fut isolé intermédiairement par ISTVAN SIMONYI et Coll., Magyar Kém. Folyoirat 62, 76-9 (1956). Ces auteurs ont préparé et recristallisé ce produit dans l'eau avant de le transformer en parahydroxybenzaldéhyde par l'acide sulfhydrique à 20 % dans l'eau, mais ils n'ont ni déterminé ses caractéristiques physiques, ni obtenu ce produit cristallisé avec deux molécules d'eau, à l'état pur permettant de se dispenser d'avoir recours à des étapes ultérieures de purification du parahydroxybenzaldéhyde résultant.

De même il est à noter que le paraformylphénolate de sodium n'a jamais été isolé à l'état cristallisé pur avec 2 molécules d'eau, malgré qu'il soit abondamment utilisé, particulièrement en solution aqueuse. En solution dans ce dernier solvant, L. A. COHEN, J. Org. Chem. 22, 1333-5 (1957) a déterminé son spectre d'absorption ultraviolet : max 330 nm log $\varepsilon = 3{,}9$.

K. K. SATPATHY et Coll., J. Indian Chem. Soc. 49, 615-20 (1972) ont étudié le comportement du nitro-3 chloro-4 benzaldéhyde vis-à-vis du paraformylphénolate de sodium sec obtenu en traitant du parahydroxybenzaldéhyde pur par la quantité stœchiométrique d'éthylate de sodium dans l'éthanol, puis en filtrant, lavant à l'éthanol et en séchant sous vide à poids constant à 100-110 °C, le précipité obtenu ; mais aucune constante physique n'est donnée.

Or, la Demanderesse a découvert fortuitement qu'on pouvait à partir du phénol et de l'acide glyoxylique en milieu aqueux sodé, accéder au paraformylphénolate de sodium cristallisé avec 2 molécules d'eau.

Selon l'invention, sans isolement du produit intermédiaire et avec utilisation du même milieu réactionnel, le procédé consiste, dans une première étape, à condenser l'acide glyoxilique en solution aqueuse sur un excès de phénol dans l'eau en présence d'hydroxyde de sodium, puis à soumettre, dans une seconde étape, la solution aqueuse précédente neutralisée et débarrassée du phénol non transformé soit par entraînement à la vapeur d'eau, soit par extraction avec un solvant organique non miscible à l'eau, à une dégradation décarboxylante oxydante catalytique en milieu alcalin sodique et enfin, dans une troisième étape, à isoler le paraformylphénolate de sodium cristallisé avec 2 molécules d'eau, après élimination du catalyseur par filtration, suivie d'une concentration à chaud du filtrat et essorage et séchage, à 20 °C sous vide, du précipité cristallisé obtenu par abandon à la température ambiante de cette solution aqueuse concentrée.

Plus précisément, ce procédé consiste à condenser entre 30 °C et 100 °C, avantageusement entre 70 °C et 85 °C, sous atmosphère inerte, 1 mole d'acide glyoxylique à 50 % dans l'eau avec 2 ou 3 moles de phénol dans environ 2 litres d'eau contenant 2 à 3 moles d'hydroxyde de sodium pendant 30 à 70 minutes, à neutraliser la solution ainsi obtenue à pH = 6,5 ± 0,3 par de l'acide sulfurique à 50 % dans l'eau, à extraire ensuite le phénol non transformé avec le dichloro-1,2 éthane, puis à soumettre la solution aqueuse alcalinisée avec 3 à 4 moles d'hydroxyde de sodium à une dégradation oxydante décarboxylante catalytique à une température ⩽ 140 °C et sous une pression d'oxygène ⩽ 6 bars en présence de sulfate cuivrique seul ou en mélange avec d'autres sels de métaux nobles du groupe VIII du tableau de Mendeleïev pendant 0,1 à 5 heures, à concentrer à chaud à 60 % plus ou moins 10 % de son poids initial la suspension aqueuse filtrée et enfin à essorer puis à sécher à poids constant sous vide à 20 °C, en présence d'hydroxyde de potassium en pastilles, le précipité cristallisé formé par abandon de ce concentrat à la température ambiante pendant quelques heures.

Le paraformylphénolate de sodium cristallisé avec 2 molécules d'eau ainsi obtenu, se présente sous forme de plaquettes incolores, hydroscopiques possédant un point de fusion instantané de 122 ± 3 °C avec décomposition. Son analyse montre qu'il est pur et qu'il contient deux molécules d'eau de cristallisation. Selon les conditions de séchage, il perd plus ou moins complètement son eau de cristallisation. Le paraformylphénolate de sodium cristallisé avec 2 molécules d'eau, ne perd pas son eau de cristallisation lorsqu'il est séché à 20 °C à poids constant sous un vide de 1 mm de mercure en présence d'hydroxyde de potassium en pastilles. Par contre, dans les mêmes conditions mais à 100 °C, on obtient le paraformylphénolate de sodium anhydre qui se présente sous forme de plaquettes légèrement jaunes, très hygroscopiques, devenant incolores par absorption d'eau, possédant un point de fusion > 250 °C.

Le paraformylphénolate de sodium cristallisé avec 2 molécules d'eau présente une solubilité aqueuse à 20 °C de 0,0916 mole dans 100 g d'eau. Contrairement à l'art antérieur, l'acidification à pH = 1 d'une telle solution aqueuse fournit quantitativement le parahydroxybenzaldéhyde cristallisé pur, de

2

point de fusion 116-117 °C, sans autres opérations de purification, ce qui prouve l'intérêt du produit selon l'invention.

On peut toutefois signaler que, selon un procédé préférentiel, pour éviter d'une part la présence éventuelle de sels de sodium corrosifs ou peu solubles dans l'eau tels que le chlorure de sodium ou le sulfate de sodium et d'autre part une perte de parahydroxybenzaldéhyde du fait de sa légère solubilité aqueuse : 0,0113 mole dans 100 g d'eau à 30,5 °C, on préfère effectuer cette transformation à chaud avec la quantité stœchiométrique d'acide acétique en solution dans 10 volumes d'eau environ. On isole ainsi après filtration de la suspension aqueuse obtenue, refroidie vers 5 °C et séchage sous vide à poids constant à 60 °C, le parahydroxy-benzaldéhyde pur, cristallisé, possédant un point de fusion de 116-117 °C, avec un rendement de 100 % de la théorie calculée par rapport au paraformylphénolate de sodium mis en œuvre.

L'invention a aussi pour objet l'application du paraformylphénolate de sodium cristallisé avec 2 molécules d'eau à la fabrication industrielle du paraanisaldéhyde.

Le paraanisaldéhyde est une essence très utilisée en parfumerie. Son emploi dans des formulations sophistiquées, exige un arôme très pur et, en particulier, l'absence de tout produit secondaire même à des concentrations de l'ordre du ppm qui altérerait son odeur.

Il est connu d'obtenir le paraanisaldéhyde par méthylation du groupe hydroxyle phénolique du parahydroxybenzaldéhyde soit à l'aide de l'iodure de méthyle selon F. Tiemann & Coll., Ber. *10*, 63 (1877), soit par l'hydroxyde de triméthylphénylammonium selon W. M. Rodionow & Coll., Arch. Pharm. *266*, 119 (1928), soit par le paratoluènesulfonate de méthyle selon S. J. Kanewskaja, Arch. Pharm. *271*, 466 (1933), soit enfin par le sulfate de diméthyle selon V. Avramenko & Coll., Khim.-Farm. Zh., *2*, 18-20 (1968) et FR 2 321 472 ou selon une variante en présence d'un solvant polaire aprotique tel que le diméthylforma-mide selon M. Pailler & Coll., Monatsch. Chem. *99*, 103 (1968).

Or, la Demanderesse a découvert qu'elle pouvait accéder au paraanisaldéhyde avec un rendement sensiblement quantitatif en méthylant par le sulfate de diméthyle en léger excès, le paraformylphénolate de sodium cristallisé avec 2 molécules d'eau, au sein d'un solvant organique non miscible à l'eau. Ainsi le fait de pouvoir disposer du paraformylphénolate de sodium cristallisé avec deux molécules d'eau, autorise sa méthylation dans un solvant organique sans l'utilisation d'agents alcalins et, en particulier, l'emploi de solvants aromatiques non polaires tels que le benzène ou le toluène qui sont industriellement économiques et qui sont connus comme étant peu favorables à la O-alkylation des phénols.

Selon l'invention, le paraformylphénolate de sodium cristallisé avec 2 molécules d'eau, est mis en suspension sous agitation dans environ 3 volumes de toluène, puis traité vers 60 °C pendant quelques heures avec un léger excès ≤ à 20 % de sulfate de diméthyle ; la suspension est ensuite diluée avec environ 2 volumes d'eau, décantée et la phase organique débarrassée du toluène par évaporation sous vide est soumise à une rectification sous vide. On isole ainsi le paraanisaldéhyde sous forme d'un liquide incolore avec un rendement supérieur à 95 % de la théorie calculée par rapport au paraformylphénolate de sodium mis en œuvre.

Selon un mode opératoire préférentiel, le paraformylphénolate de sodium recristallisé brut humide, non séché, est mis en suspension dans environ 3 volumes de toluène. Cette suspension est ensuite chauffée au reflux jusqu'à élimination par distillation azéotropique d'environ la moitié de l'eau présente, puis la suspension refroidie vers 60 °C est traitée sous agitation avec un léger excès ≤ à 20 % de sulfate de diméthyle introduit goutte à goutte en une heure environ ; le chauffage et l'agitation sont poursuivis ensuite pendant 2 à 3 heures, avant de diluer cette suspension avec 2 volumes d'eau environ. Après décantation, lavage au toluène de la phase aqueuse écartée, puis lavage à l'eau des phases organiques réunies, le résidu huileux obtenu après élimination du toluène par évaporation sous vide de la phase organique est rectifié sous vide. On isole ainsi le paraanisaldéhyde liquide, possédant un point d'ébullition sous 30 mm de mercure de 138-141 °C avec un rendement supérieur à 95 % de la théorie calculée par rapport au paraformylphénolate de sodium mis en œuvre.

Les exemples suivants sont donnés à titre illustratif et nullement limitatif de l'invention.

Exemple 1

On chauffe 30 minutes à 82 °C, sous agitation et en atmosphère d'azote, une solution de 296 g (2 moles) d'acide glyoxylique à 50 % dans l'eau, 564 g (6 moles) de phénol et 160 g (4 moles) de soude en pastilles dans 3,6 litres d'eau. Après refroidissement à 40 °C, on amène la solution réactionnelle à pH 6,5 par de l'acide sulfurique à 50 % dans l'eau, puis après refroidissement à la température ambiante, on extrait le phénol non transformé au moyen de dichloro-1,2-éthane ; on isole ainsi, après élimination du solvant, 376 g (4 moles) de phénol. La solution aqueuse alcalinisée avec 8 moles d'hydroxyde de sodium est chauffée 5 heures à 120 °C sous une pression de 4 bars d'oxygène en présence de 3,5 g de sulfate cuivrique pentahydraté. Après refroidissement et mise à la pression ambiante, la suspension obtenue, filtrée, est concentrée à 55 % environ de son poids initial sous vide à une température inférieure à 85 °C, puis abandonnée quelques heures à la cristallisation à température ambiante. Le précipité cristallisé est ensuite essoré, lavé à l'eau par empâtage et séché à 20 °C sous un vide de 1 mm de mercure à poids constant en présence d'hydroxyde de potassium en pastilles. On isole ainsi 216,16 g (1,2 mole) de paraformylphénolate de sodium cristallisé avec 2 molécules d'eau en plaquettes incolores, possédant un point de fusion de 122 ± 3 °C avec décomposition, soit un rendement de 60 % par rapport à l'acide glyoxylique mis en œuvre. Ce produit peut être recristallisé par chaud et froid dans 0,75 volume d'eau avec un rendement de 60 % pour le premier jet. Le point de fusion n'est pas modifié.

Micro-analyse : produit très hygroscopique.

|  |  | C % | H % | H$_2$O %** | Cendres* |
|---|---|---|---|---|---|
| C$_7$H$_5$O$_2$Na, $_2$H$_2$O | Calculés | 46,67 | 5,03 | 20,0 | 39,43 |
| PM = 180,14 | Trouvés | 46,4 | 5,1 | 20,2 | 38,9 |

* Cendres sulfuriques
** déterminé par perte à l'étuve à 100 °C.

Solubilité dans 100 g d'eau à 20 °C : 0,0916 mole
Analyses physiques :
RMN du proton dans le DMSO deutéré (TMS référence interne)

| δ = 9,12 ppm | singulet | 1H | H (CHO) |
|---|---|---|---|
| δ = 6,65 ppm | massif | 4H | aromatique |

CPV (effectuée sur le phénol libre obtenu par acidification) absence d'isomère ortho.

### Exemple 2

On opère comme dans l'exemple 1 mais on effectue la dégradation oxydante décarboxylante à 110 °C sous une pression d'oxygène de 6 bars durant 4 heures. On isole ainsi le paraformylphénolate de sodium cristallisé avec 2 molécules d'eau, possédant un point de fusion de 122 ± 3 °C avec un rendement de 57,6 % par rapport à l'acide glyoxylique mis en œuvre.

### Exemple 3

On opère comme dans l'exemple 1 mais on effectue la dégradation oxydante décarboxylante en 3 heures en présence de 3 g de sulfate cuivrique, pentahydraté et de 3 g de chlorure ferrique. On isole ainsi le paraformylphénolate de sodium cristallisé avec 2 molécules d'eau, possédant un point de fusion 122 ± 3 °C avec un rendement de 56,2 % par rapport à l'acide glyoxylique mis en œuvre.

### Exemple 4

On opère comme dans l'exemple 3 mais on utilise comme catalyseur de dégradation oxydante décarboxylante 3 g de sulfate cuivrique pentahydraté et 3 g d'acétate de cobalt tétrahydraté. On isole ainsi le paraformylphénolate de sodium cristallisé avec 2 molécules d'eau avec un rendement de 60 % par rapport à l'acide glyoxylique mis en œuvre.

### Exemple 5

On chauffe 30 minutes à 100 °C, sous agitation et en atmosphère d'azote, une solution de 296 g (2 moles) d'acide glyoxylique à 50 % dans l'eau, 564 g (6 moles) de phénol et 160 g (4 moles) de soude en pastilles dans 3,6 litres d'eau. Puis on opère comme dans l'exemple 1. On isole ainsi le paraformylphénolate de sodium cristallisé avec 2 molécules d'eau possédant un point de fusion de 122 ± 3 °C, avec un rendement de 56,47 % par rapport à l'acide glyoxylique mis en œuvre.

### Exemple 6

On chauffe 1 heure à 50 °C, sous agitation et en atmosphère d'azote, une solution de 296 g (2 moles) d'acide glyoxylique à 50 % dans l'eau, 564 g (6 moles) de phénol et 200 g (5 moles) de soude en pastilles dans 3,6 litres d'eau. Puis on opère comme dans l'exemple 1. On isole ainsi le paraformylphénolate de sodium cristallisé avec 2 molécules d'eau possédant un point de fusion de 122 ± 3 °C, avec un rendement de 61,2 % par rapport à l'acide glyoxylique mis en œuvre.

### Exemple 7

On chauffe en 30 minutes de 30 à 85 °C, puis on refroidit en 30 minutes à 40 °C, sous agitation et en atmosphère d'azote, une solution de 296 g (2 moles) d'acide glyoxylique à 50 % dans l'eau, 564 g (6 moles) de phénol et 180 g (4,5 moles) de soude en pastilles dans 3,6 litres d'eau. Puis on opère comme dans l'exemple 1.

On isole ainsi le paraformylphénolate de sodium cristallisé avec 2 molécules d'eau possédant un point de fusion de 122 ± 3 °C, avec un rendement de 63,5 % par rapport à l'acide glyoxylique mis en œuvre.

## Exemple 8

On chauffe 30 minutes à 70 °C, sous agitation et en atmosphère d'azote, une solution de 296 g (2 moles) d'acide glyoxylique à 50 % dans l'eau, 564 g (6 moles) de phénol et 160 g (4 moles) de soude en pastilles dans 3,6 litres d'eau. Puis on opère comme dans l'exemple 1. On isole ainsi le paraformylphénolate de sodium cristallisé avec 2 molécules d'eau, possédant un point de fusion de 122 ± 3 °C, avec un rendement de 65,5 % par rapport à l'acide glyoxylique mis en œuvre.

## Exemple 9

On traite 0,225 mole (40,53 g) de paraformylphénolate de sodium cristallisé avec 2 molécules d'eau par 150 g d'une solution aqueuse contenant 0,225 mole d'acide acétique. On chauffe cette suspension vers 80 °C, puis la solution obtenue est refroidie à 5 ± 2 °C pendant quelques heures. Le précipité cristallisé ainsi obtenu est essoré, lavé à l'eau puis séché à poids constant sous 30 mm de mercure à 60 °C. On isole ainsi 0,225 mole (27,4 g) de parahydroxybenzaldéhyde cristallisé possédant un point de fusion de 116-117 °C, soit un rendement quantitatif de la théorie calculée par rapport au paraformylphénolate de sodium mis en œuvre.

Microanalyse

|  |  | C % | H % |
|---|---|---|---|
| $C_7H_6O_2$ | Calculés | 68,84 | 4,95 |
| PM = 122,12 | Trouvés | 68,6 | 4,7 |

## Exemple 10

On traite à 60 °C ± 3 °C, une suspension agitée de 2 moles (360 g) de paraformylphénolate de sodium cristallisé avec 2 molécules d'eau dans 3 volumes de toluène par 2,4 moles (302,7 g) de sulfate de diméthyle introduit lentement en une heure environ. On poursuit le chauffage à 60 °C et l'agitation durant encore 150 minutes après la fin de l'introduction, puis on dilue le milieu réactionnel chaud avec 450 cm³ d'eau. La suspension obtenue refroidie est décantée, la phase aqueuse est lavée au toluène, et les phases organiques réunies sont lavées à l'eau, séchées, filtrées, et enfin concentrées sous vide à 60 °C pour éliminer le toluène. Le résidu huileux obtenu est rectifié sous un vide de 30 mm de mercure. On recueille ainsi 1,94 mole (264,11 g) de paraanisaldéhyde sous forme d'huile incolore distillant sous 30 mm de mercure à 138-141 °C, soit un rendement de 97 % de la théorie calculée par rapport au paraformylphénolate de sodium mis en œuvre.

Microanalyse

|  |  | C % | H % |
|---|---|---|---|
| $C_8H_8O_2$ | Calculés | 70,58 | 5,92 |
| PM = 136,13 | Trouvés | 70,8 | 6,0 |

Dosage fonctionnel : —CHO (NH₂OH, HCl) : 99,8 ± 1 %

## Exemple 11

On chauffe au reflux dans 3 volumes de toluène avec distillation azéotropique de l'eau, 576 g de paraformylphénolate de sodium cristallisé avec 2 molécules d'eau non séché contenant 38 % d'eau (soit 1,98 mole de paraformylphénolate de sodium cristallisé avec 2 molécules d'eau et 219 g d'eau d'hydratation). Après avoir recueilli 120 g d'eau dans le distillat, on abaisse la température du milieu réactionnel à 60 °C et on ajoute sous agitation en 1 heure environ 265 g (2,1 moles) de sulfate de diméthyle, puis on poursuit le chauffage à 60 °C et l'agitation pendant 150 minutes après la fin de l'introduction. La suspension obtenue est ensuite traitée comme dans l'exemple 9. On isole ainsi après distillation 261 g (1,92 mole) de paraanisaldéhyde possédant un point d'ébullition sous 30 mm de mercure de 138-141 °C, soit un rendement de 97 % de la théorie calculée par rapport au paraformylphénolate de sodium mis en œuvre.

Il va de soi que la présente invention n'a été décrite qu'à titre purement explicatif et nullement limitatif et que toute modification utile pourra y être apportée sans sortir de son cadre tel que défini par les revendications ci-après.

**Revendications**

1. Le paraformylphénolate de sodium cristallisé avec 2 molécules d'eau.

2. Procédé de fabrication du paraformylphénolate de sodium cristallisé, caractérisé par le fait :

   a) que l'on condense dans l'eau en présence d'hydroxyde de sodium à une température comprise entre 30 °C et 100 °C, l'acide glyoxylique sur un excès de phénol ;

   b) que la solution aqueuse ainsi obtenue, neutralisée puis débarrassée du phénol non transformé soit par entraînement à la vapeur d'eau, soit par extraction avec un solvant organique non miscible à l'eau, est soumise à chaud, sous pression d'oxygène, à une dégradation oxydante décarboxylante en présence d'hydroxyde de sodium et d'un catalyseur à base de sels cuivriques ;

   c) que la solution aqueuse obtenue après élimination du catalyseur par filtration, puis concentration à chaud sous vide à 60 % plus ou moins 10 % de son volume initial est abandonnée à la cristallisation ;

   d) que le précipité ainsi obtenu est essoré, lavé puis séché à poids constant sous vide de 1 mm de mercure à 20 °C, pour obtenir le paraformylphénolate de sodium cristallisé avec 2 molécules d'eau ou séché à poids constant sous vide de 1 mm de mercure à 100 °C pour l'obtenir anhydre.

3. Procédé selon la revendication 2, caractérisé par le fait que la condensation de l'acide glyoxylique sur le phénol s'effectue à une température comprise entre 70 °C et 85 °C.

4. Procédé selon la revendication 2 ou 3, caractérisé par le fait que l'on effectue la condensation d'une mole d'acide glyoxylique en solution aqueuse sur un excès de 2 à 3 moles de phénol.

5. Procédé selon l'une quelconque des revendications 2 à 4, caractérisé par le fait que l'on condense une mole d'acide glyoxylique en solution aqueuse sur un excès de phénol en présence de 2 à 3 moles d'hydroxyde de sodium.

6. Procédé selon l'une quelconque des revendications 2 à 5, caractérisé par le fait que la dégradation oxydante décarboxylante est effectuée sur la solution aqueuse de condensation de l'acide glyoxylique sur le phénol débarrassé du phénol non transformé.

7. Procédé selon l'une quelconque des revendications 2 à 6, caractérisé par le fait que la dégradation oxydante décarboxylante est effectuée à une température comprise entre 90 °C et 140 °C.

8. Procédé selon l'une quelconque des revendications 2 à 7, caractérisé par le fait que la dégradation oxydante décarboxylante est effectuée sous pression d'oxygène inférieure ou égale à 6 bars.

9. Procédé selon l'une quelconque des revendications 2 à 8, caractérisé par le fait que la dégradation oxydante décarboxylante est effectuée en présence de 2 à 6 moles d'hydroxyde de sodium par rapport à l'acide glyoxylique mis en œuvre.

10. Procédé selon la revendication 2, caractérisé par le fait que la dégradation oxydante décarboxylante est effectuée en présence de sels cuivriques et de sels choisis parmi les métaux du groupe VIII du tableau de Mendeleiev.

11. Application du paraformylphénolate de sodium cristallisé avec 2 molécules d'eau à la préparation du paraanisaldéhyde.

12. Application selon la revendication 11, caractérisé par le fait que l'on méthyle ledit paraformylphénolate de sodium cristallisé avec 2 molécules d'eau par le sulfate de diméthyle au reflux d'un solvant organique.

13. Application selon la revendication 12, caractérisée par le fait que le solvant organique est le toluène.

14. Application selon la revendication 12, caractérisée par le fait que l'on méthyle le paraformylphénolate de sodium cristallisé avec 2 molécules d'eau, brut, humide, non séché, par le sulfate de diméthyle en léger excès au reflux du toluène après élimination partielle de l'eau présente par distillation azéotropique.

**Claims**

1. Crystallized sodium paraformylphenolate with two molecules of water.

2. A process of preparation of crystallized sodium paraformylphenolate characterized in that :

   a) glyoxylic acid is condensed in water in the presence of sodium hydroxide at a temperature of between 30 and 100 °C on an excess of phenol ;

   b) the so obtained aqueous solution, neutralized and then freed from the untransformed phenol either by water vapor driving or by extraction with a water immiscible organic solvent is submitted in the hot state under an oxygen pressure to an oxidizing decarboxylating degradation in the presence of sodium hydroxide and a catalyst based on cupric salts ;

    c) the aqueous solution obtained after elimination of the catalyst by filtration and then concentration in the hot state under vacuum to 60 % plus or minus 10 %, of its initial volume is left to crystallize ;

    d) the so obtained precipitate is spin dried, washed and then dried to constant weight under a vacuum of 1 mm of mercury at 20 °C to obtain the crystallized sodium paraformylphenolate with 2 molecules of water or dried to constant weight under a vacuum of 1 mm of mercury at 100 °C to obtain it anhydrous.

3. A process as claimed in claim 2, characterized in that the condensation of the glyoxylic acid on phenol is effected at a temperature of from 70 to 85 °C.

4. A process according to any of claims 2 or 3, characterized by effecting condensation of one mole of glyoxylic acid in aqueous solution on an excess of 2 to 3 moles of phenol.

5. A process according to any of claims 2 to 4, characterized in that one mole of glyoxylic acid is condensed in aqueous solution on an excess of phenol in the presence of 2 to 3 moles of sodium hydroxide.

6. A process according to any of claims 2 to 5, characterized by effecting the oxidizing decarboxylating degradation on the aqueous solution of condensation of glyoxylic acid on phenol, as freed from the untransformed phenol.

7. A process according to any of claims 2 to 6, characterized by effecting the oxidizing decarboxylating degradation at a temperature of from 90 to 140 °C.

8. A process according to any of claims 2 to 7, characterized by effecting the oxidizing decarboxylating degradation under an oxygen pressure lower than, or equal to, 6 bars.

9. A process according to any of claims 2 to 8, characterized by effecting the oxidizing decarboxylating degradation in the presence of 2 to 6 moles of sodium hydroxide with respect to the glyoxylic acid.

10. A process according to claim 2, characterized by effecting the oxidizing decarboxylating degradation in the presence of cupric salts and salts selected among the metals from the group VIII of Mendeleev's Table.

11. The application of the crystallized sodium paraformylphenolate with 2 molecules of water for the preparation of paraanisaldehide.

12. An application according to claim 11, characterized by methylating said crystallized sodium paraformylphenolate with 2 molecules of water by dimethyl sulfate to the reflux of an organic salt.

13. An application according to claim 12, characterized in that the organic solvent is toluene.

14. An application according to claim 12, characterized by methylating the crystallized sodium paraformylphenolate with 2 molecules of water, which is raw, moist, not dried, by dimethyl sulfate in slight excess to the reflux of toluene after partial elimination of the water present by azeotropic distillation.


**Ansprüche**

1. Natriumparaformylphenolat, kristallisiert mit 2 Mol Wasser.

2. Verfahren zur Herstellung von kristallisiertem Natriumparaformylphenolat, dadurch gekennzeichnet :

    a) daß man in Wasser in Gegenwart von Natriumhydroxyd bei einer Temperatur zwischen 30 °C und 100 °C Glyoxylsäure mit einem Überschuß von Phenol kondensiert ;

    b) daß die so erhaltene wässrige Lösung, nach Neutralisieren und anschließender Entfernung von nicht umgesetztem Phenol durch Abschleppen mit Wasserdampf oder durch Extraktion mit einem organischen, mit Wasser nicht mischbaren Lösungsmittel, in der Hitze unter Sauerstoffdruck einem oxydativen dekarboxylierenden Abbau in Gegenwart von Natriumhydroxyd und einem Katalysator auf der Basis von Cuprisalzen unterworfen wird ;

    c) daß die nach Beseitigung des Katalysators durch Filtration und anschließende Konzentration in der Hitze unter Vakuum auf 60 % + oder −10 % des Ausgangsvolumens erhaltene wässrige Lösung zur Kristallisation stehengelassen wird ;

    d) daß der so erhaltene Niederschlag abgesaugt, gewaschen und dann bis zum konstanten Gewicht unter Vakuum von 1 mm Hg bei 20 °C getrocknet wird um mit 2 Mol Wasser kristallisiertes Natriumparaformylphenolat zur erhalten oder zur Gewichtskonstanz unter Vakuum von 1 mm Hg bei 100 °C getrocknet wird um es wasserfrei zu erhalten.

3. Verfahren nach Anspruch 2, dadurch gekennzeichnet, daß die Kondensation von Glyoxylsäure mit Phenol bei einer Temperatur zwischen 70 °C und 85 °C durchgeführt wird.

4. Verfahren nach Anspruch 2 oder 3, dadurch gekennzeichnet, daß die Kondensation von 1 Mol Glyoxylsäure in wässriger Lösung mit einem Überschuß von 2 bis 3 Mol Phenol durchgeführt wird.

5. Verfahren nach irgendeinem der Ansprüche 2 bis 4, dadurch gekennzeichnet, daß man 1 Mol Glyoxylsäure in wässriger Lösung mit einem Überschuß von Phenol in Gegenwart von 2 bis 3 Mol Natriumhydroxyd kondensiert.

6. Verfahren nach irgendeinem der Ansprüche 2 bis 5, dadurch gekennzeichnet, daß der oxydierende dekarboxylierende Abbau an der wässrigen Lösung der Kondensation von Glyoxylsäure mit Phenol, die von nicht umgesetztem Phenol befreit ist, durchgeführt wird.

7. Verfahren nach irgendeinem der Ansprüche 2 bis 6, dadurch gekennzeichnet, daß der oxydierende dekarboxylierende Abbau bei einer Temperatur zwischen 90 °C und 140 °C durchgeführt wird.

8. Verfahren nach irgendeinem der Ansprüche 2 bis 7, dadurch gekennzeichnet, daß der oxydierende dekarboxylierende Abbau unter einem Sauerstoffdruck von weniger oder gleich 6 bar durchgeführt wird.

9. Verfahren nach irgendeinem der Ansprüche 2 bis 8, dadurch gekennzeichnet, daß der oxydierende dekarboxylierende Abbau in Gegenwart von 2 bis 6 Mol Natriumhydroxyd, bezogen auf eingesetzte Glyoxylsäure, durchgeführt wird.

10. Verfahren nach Anspruch 2, dadurch gekennzeichnet, daß der oxydierende dekarboxylierende Abbau in Gegenwart von Cuprisalzen und von Salzen der Metalle der Gruppe VIII des Periodensystems nach Mendelejeff durchgeführt wird.

11. Anwendung von mit 2 Mol Wasser kristallisiertem Natriumparaformylphenolat zur Herstellung von Paraanisaldehyd.

12. Anwendung nach Anspruch 11, dadurch gekennzeichnet, daß man dieses mit 2 Mol Wasser kristallisierte Natriumparaformylphenolat mit Dimethylsulfat unter Rückfluß eines organischen Lösungsmittels methyliert.

13. Anwendung nach Anspruch 12, dadurch gekennzeichnet, daß das organische Lösungsmittel Toluol ist.

14. Anwendung nach Anspruch 12, dadurch gekennzeichnet, daß man das mit 2 Mol Wasser kristallisierte Natriumparaformylphenolat in rohem, feuchtem, nicht getrocknetem Zustand mit Dimethylsulfat in leichtem Überschuß unter Toluol-Rückfluß nach teilweiser Beseitigung des vorhandenen Wassers durch azeotrope Destilation methyliert.